# EUROPEAN PATENT APPLICATION

(11) **EP 4 282 948 A1**
(43) Date of publication of application: **29.11.2023**
(21) Application number: 22175840.2
(22) Date of filing: 27.05.2022
(51) Int. Cl.: C12M 3/06, B01L 3/00

(54) **CELL CULTURE APPARATUS AND METHOD**

(71) Applicant: Finnadvance Oy, 90220 Oulu (FI)
(72) Inventor: Singh, Prateek, 90220 Oulu (FI); Nguyen, Hoang Tuan, 90220 Oulu (FI)
(74) Representative: Papula Oy

(57) **Abstract**

Disclosed is a cell culture apparatus and a method. The apparatus comprises one or more cell culture modules. The one or more cell culture modules comprise a first culture medium reservoir, a second culture medium reservoir, a first open-top well configured for cell cultivation, a second open-top well and a microfluidic channel configured to provide a fluid connection from the first culture medium reservoir to the second culture medium reservoir through the first open-top well and the second open-top well. The second open-top well is configured to receive a flow restrictor to at least partially block the fluid connection through the second open-top well.

## Description

### FIELD

The present disclosure relates to biotechnology. In particular, the present disclosure relates to cell culture.

### BACKGROUND

Cell culture or cell cultivation involves growing cells of desired type(s) outside a living body under controlled in vitro conditions to sustain cell growth and viability. The cell culture is widely used in different biotechnology branches, including cell biology, tissue engineering, biomedical engineering, cell differentiation studies, cell-based biosensors, cell-cell interaction, cell-signaling, cell-migration, physiological and pathophysiological studies, etc.

Environmental conditions created for cultured cells should resemble as closely as possible the conditions experienced by the same cells in vivo. This may be done by performing the cell culture in large vessels, such as dishes, spinner and shaker flasks, etc. However, the cell culture conditions provided by these vessels do not truly represent the in vivo environment of the cells being cultured. Moreover, since these vessels have a large volume, they consume significant amounts of reagents, culture media, chemicals, etc., thereby making it difficult to control and/or alter the cell culture conditions.

With the advent of microfluidics, new devices and methods configured to cultivate various types of cells, like adherent and non-adherent, under uniform and controlled in vitro conditions have been developed. Unlike the conventional cell culture methods based on the above-mentioned vessels, microfluidic cell culture methods may provide continuous nutrient (culture fluid or medium) supply, waste removal, flexibility of schedules, and high automation capability. The less consumption of fluids, their low volumes and therefore the reduced time and cost of cell cultivation make these microfluidic methods particularly interesting for cell-based assays. Some main goals of microfluidic cell culture devices are to mimic closely in vivo cellular microenvironments and to maintain simplicity for reproducible results.

However, the limiting factors of the current microfluidic cell culture devices are their low throughput and incompatibility with available liquid handling systems and imaging systems due to the discrete arrangement and limited number of microfluidic channels. Furthermore, fluid flow control in the current microfluidic cell culture devices is often limited to one method and not flexible enough to adapt to different resource settings and different cell-based assays.

### SUMMARY

An objective is to alleviate the disadvantages mentioned above. In particular, it is an objective to provide a cell culture apparatus with improved cultivation conditions. Another objective is to provide a technical solution that enables high-throughput microfluidic cell culture.

According to a first aspect, a cell culture apparatus comprises one or more cell culture modules. The one or more cell culture modules comprise a first and a second culture medium reservoir, a first open-top well configured for cell cultivation and a second open-top well, which may also be configured for cell cultivation and a microfluidic channel configured to provide a fluid connection from the first culture medium reservoir to the second culture medium reservoir through the first open-top well and the second open-top well. The second open-top well is configured to receive a flow restrictor to at least partially block the fluid connection through the second open-top well.

The cell culture apparatus allows cultivation of cells of the same or different type in the first and, optionally, the second open-top well. This can be done under uniform and controlled in vitro conditions. However, the second open-top well can also be utilized for restriction of flow through the microfluidic channel with the flow restrictor. This allows for variable flow rates in a single cell culture module.

In an embodiment, apparatus is configured for the flow restrictor, in a blocking position, to completely block the fluid connection through the second open-top well and, in a connecting position, facilitate the fluid connection through the second open-top well. This allows the fluid connection to be completely blocked.

In an embodiment, apparatus is configured for the flow restrictor, in a first connecting position, to facilitate the fluid connection through the second open-top well with a first flow rate and, in a second connecting position, facilitate the fluid connection through the second open-top well with a second flow rate, the second flow rate being smaller than the first. This allows the flow rate for the fluid connection to be controlled with the flow restrictor.

In an embodiment, the flow restrictor is a plunger. The flow restrictor can thus be moved within the second open-top well for adjusting flow with translational movement of the flow restrictor. This allows ease of use and manufacture as the flow restrictor can be provided as a plug for the open-top well. In an embodiment, the apparatus is configured for the plunger to be moved perpendicularly with respect to the fluid connection through the second open-top well for at least partially blocking the fluid connection. This allows a particularly expedient configuration for the second open-top well, which can also be utilized for other open-top wells of the cell culture module(s), including the first open-top well. This provides one example of a configuration, where the open-top wells can easily be made interchangeable so that a single open-top well can be used both for cell cultivation and for flow restriction, although it is noted that other configurations allow this as well.

In an embodiment, the flow restrictor has a rotation axis and, with the rotation axis perpendicular to the fluid connection through the second open-top well, is configured to rotate about the rotation axis between a first connecting position and a second connecting position and/or a blocking position for the fluid connection. This allows the flow restrictor to switch between different flow-adjusting positions with rotational movement, i.e., without translational movement if so desired.

In an embodiment, the one or more cell culture modules comprises a third open-top well configured for cell cultivation. The third open-top well may be positioned between the first culture medium reservoir and the second culture medium reservoir for the fluid connection. This allows for multiple cell cultures with fluid connection between each other at the same time, e.g. in the first and third open-top wells. Consequently, one open-well may be used for cultivation of cells of a first type and another open-top well for cultivation of cells of a second, different type, e.g. for simulating different types of tissues simulated and their interaction through the fluid connection. The module as a whole then allows facilitating the interaction of different types of cell cultures through the microfluidic channel. The second open-top well may be positioned between the first open-top well and the third open-top well for the fluid connection, allowing the fluid connection between the first open-top well and the third open-top well to be controlled with the flow restrictor.

In a further embodiment, the one or more cell culture modules comprises a fourth open-top well, which may also be configured for cell cultivation. The second open-top well and the fourth open-top well may be positioned between the first open-top well and the third open-top well for the fluid connection. The fourth open-top well may be configured to receive the flow restrictor to at least partially block the fluid connection through the fourth open-top well. In this configuration, the flow restrictor can be interchangeable between the second and the fourth open-top well. Alternatively, the fourth open-top well may be configured to receive a second flow restrictor to at least partially block the fluid connection through the fourth open-top well. In this configuration, the flow restrictor and the second flow restrictor are non-interchangeable. The configuration with two open-top wells for flow restrictors allows for variable flow rates for two cell cultures in the same cell culture module.

The apparatus may comprise a first and/or a second flow restrictor, both being configured for acting as the flow restrictor for either or both of the second open-top well and the fourth open-top well. In an embodiment, the apparatus can thus be configured for the second flow restrictor to at least partially block the fluid connection through the fourth open-top well while the first flow restrictor, acting as the flow restrictor as described herein, at least partially blocks the fluid connection through the second open-top well.

In an embodiment, the bottom of the microfluidic channel is shaped to match the bottom of the flow restrictor at the location of the second open-top well for preventing fluid connection below the bottom of the flow restrictor when the flow restrictor is positioned to at least partially block the fluid connection. When the cell culture module comprises the fourth open-top well, the same may hold for the bottom of the microfluidic channel at the location of the fourth open-top well for the flow restrictor and/or the second flow restrictor.

In an embodiment, the bottom of the microfluidic channel, at least at the location of the second open-top well and, optionally, at the location of the fourth open-top well is flat.

In an embodiment, the second-open top well comprises a wall extending upward from the microfluidic channel and the apparatus is configured for the flow restrictor to be supported against the wall. This allows the flow restrictor to function as a plug for the open-top opening defined by the wall. When the cell culture module comprises the fourth open-top well, the same may hold for the wall of the fourth open-top well with respect to the flow restrictor and/or the second flow restrictor.

In an embodiment, the fluid connection between the first and the second culture medium reservoir is linear. This allows the cell culture module to be provided in a compact manner with favorable characteristics for the fluid connection.

In an embodiment, the fluid connection between the first and the second culture medium reservoir forms a meandering pattern. This allows positions for multiple cell culture modules to be optimized within the cell culture apparatus.

In an embodiment, the distance between any or all adjacent culture medium reservoirs and/or open-top wells is equal. The distance, which corresponds to the path length for the fluid connection, affects the flow profile in the microfluidic channel. For come cell types, for example brain vascular, laminar flow is advantageous, and for others, like lung, the flow can reach turbulent. Changes in the distance can thus be used to facilitate laminar and/or turbulent flow at the microfluidic channel.

According to a second aspect, a method, which can be used for facilitating cell cultivation, is provided. The method comprises providing a cell culture apparatus comprising one or more cell culture modules, the one or more cell culture modules comprising a first culture medium reservoir, a second culture medium reservoir, a first open-top well configured for cell cultivation, a second open-top well and a microfluidic channel configured to provide a fluid connection from the first culture medium reservoir to the second culture medium reservoir through the first open-top well and the second open-top well. The cell culture apparatus may be one according to the first aspect or any of its embodiments, alone or in any combination. The method also comprises inserting and/or adjusting a flow restrictor, at least, to the second open-top well for at least partially blocking the fluid connection through the second open-top well. When the cell culture module comprises also a fourth open-top well, the method may comprise inserting and/or adjusting the flow restrictor and/or the second flow restrictor to the fourth open-top well for at least partially blocking the fluid connection through the fourth open-top well.

It is to be understood that the aspects and embodiments described above may be used in any combination with each other. Several of the aspects and embodiments may be combined together to form a further embodiment of the invention.

### BRIEF DESCRIPTION OF THE DRAWINGS

The accompanying drawings, which are included to provide a further understanding and constitute a part of this specification, illustrate examples and together with the description help to explain the principles of the disclosure. In the drawings:
**Fig. 1** illustrates a cell culture apparatus according to an example,
**Fig. 2** illustrates a cell culture module according to a first example in a side view for various states,
**Fig. 3** illustrates a cell culture module according to a second example in a top-down view for various states,
**Fig. 4** illustrates a cell culture module according to a third example in a side view for various states,
**Fig. 5** illustrates a cell culture module according to a fourth example in a top-down view for various states, and
**Fig. 6** illustrates a method according to an example.

Like references are used to designate equivalent or at least functionally equivalent parts in the accompanying drawings.

### DETAILED DESCRIPTION

The detailed description provided below in connection with the appended drawings is intended as a description of examples and is not intended to represent the only forms in which the example may be constructed or utilized. However, the same or equivalent functions and structures may be accomplished by different examples.

Herein, a cell may refer to a biological cell, such as a plant cell, an animal cell (e.g., a mammalian cell), a bacterial cell, a fungal cell, or the like. A mammalian cell may be, for example, from a human, a mouse, a horse, a goat, a sheep, a cow, a primate, or the like. In particular, the cell may be a specialized cell such as a brain vascular cell or a lung cell.

Culture medium, also referred to as a growth medium, may refer to a liquid, suspension, or gel designed to support cellular growth in an artificial environment, i.e., in vitro. There are different types of culture media suitable for growing different types of cells. In general, the culture media may be broken into two primary categories: natural media and synthetic media. The natural media may refer to those that are derived from tissue extraction or animal body fluids, such as plasma, lymph, and serum. The synthetic media may refer to those created using a variety of organic and inorganic compounds. Moreover, the culture medium itself may comprise cells, bodies of cells (e.g., organoids), lipid particles, including natural or engineered (e.g., exosomal microvesicles, vacuoles, micelles or lipid particles of various design, virus particles, nanoparticles, etc.).

A cell culture apparatus may refer to an apparatus having various wells (e.g., reservoirs, chambers, etc.) connected by microchannels in which fluid, such as culture medium, will exhibit microfluidic behavior in its flow through the microchannels. Such a cell culture apparatus may also be referred to as a microfluidic chip in this technical field. The microfluidic cell culture performed by the cell culture apparatus can be generally related to cell culture, maintenance and perturbation in micro-scale fluid volumes. The reasons behind the popularity of the microfluidic cell culture are both economic and scientific. The microfluidic chips have the advantages of in vitro cell culture (high-throughput, parallel experiments, experiments may be done at the discretion of an experimenter, no need for specialized infrastructure and personnel, etc.) with in vivo like performance. For example, in mouse models, drugs are really selected for mice, not humans. By using human cells, drugs are screened for humans. Thus, using humanized microfluidic cell and tissue cultures to screen drug candidates reduces pre-clinical trial time and those drugs entering clinical testing are better suited for humans. This may reduce the probability of adverse effects and increase the chance of showing efficacy resulting in less failures in clinical trials.

Each microchannel of the cell culture apparatus can also be referred to as a microfluidic channel or as a flow channel and may correspond to a sub-millimeter-scale channel that has a hydraulic diameter below 1 mm and is used for fluidically connecting the wells, including reservoirs, of the cell culture apparatus. In other words, the microfluidic channel may generally indicate a channel configured to pass different fluids, such, for example, as culture media (e.g., cell suspensions), reagents, or gels, in micro-scale volumes. The microfluidic channel may be shaped such that it has appropriate flow characteristics (e.g., flow rates) depending on particular applications. For example, the microfluidic channel may have a rectangular, e.g., square, or rounded cross-section, as well as be straight, bended, or tilted towards a required direction.

The microfluidic cell culture may involve using a separating interface such as a porous membrane sandwiched between two flow layers within a microchannel or between a culture chamber and a microchannel for cell deposition. However, many membrane-based cell culture apparatuses known so far suffer from low throughput and incompatibility with available liquid handling systems (e.g., microtiter plate formats) and imaging systems. Furthermore, the flow control in such apparatuses is often limited to one method and not flexible enough to adapt to different resource settings. On top of that, the cell deposition on the basal (i.e., bottom) surface of separating interface is often possible in the current cell culture apparatuses only with very high cell concentrations, liquid flow control, and by inverting the cell culture apparatus.

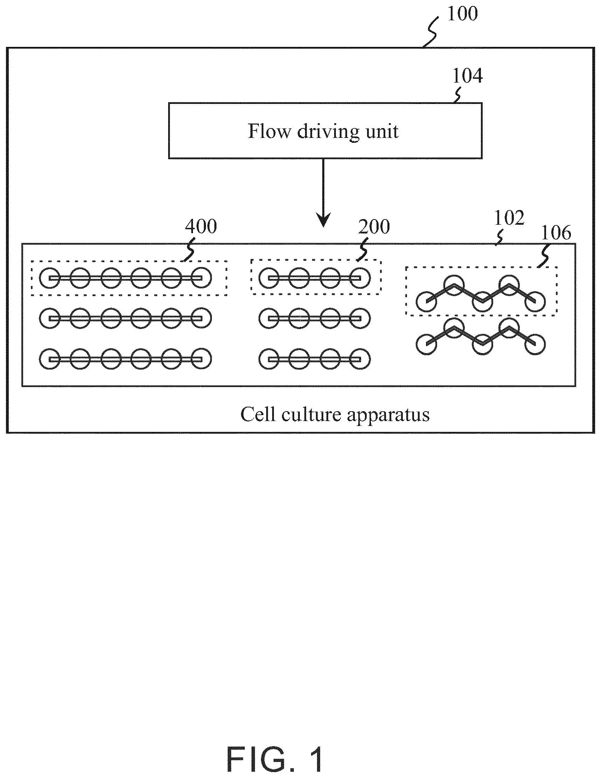

Figure 1 shows an example of a cell culture apparatus 100 (also "the apparatus"), which can be configured for microfluidic cell culture, e.g. as described above. The apparatus comprises a plurality of cell culture modules 106, 200, 400 (also "the modules"), as emphasized by the dotted lines, arranged adjacent to each other. The modules may be arranged in a regular pattern to facilitate automatized handling. They may be arranged in rows and/or columns of modules, e.g. as a matrix formation. The apparatus 100 may comprise a cell culture plate 102 on which the modules are located. The plate may be configured to fit the standard 48, 96, 128 or 258 microtiter plates, for example. For this purpose, ANSI/SLAS Microplate Standards may be followed.

The apparatus may also comprise a flow driving unit 104, which may be coupled to the cell culture plate 102. The flow driving unit can be configured to cause a culture medium to flow in each cell culture module. The flow driving unit may be a rocking platform that is configured to periodically rock the cell culture plate, or the one or more cell culture modules, thereby facilitating the flow of the culture medium. The flow driving unit may also be a pneumatic pump that is configured to supply compressed gas or pressurized air to the first and/or second culture medium reservoir in any or all of the one or more cell culture modules, thereby facilitating the flow of the culture medium. The flow driving unit may be configured to facilitate the flow of the culture medium by pipetting the culture medium from the first culture medium reservoir to the second culture medium reservoir, or vice versa, for example at regular time intervals or based on an amount of the culture medium in the first and/or the second culture medium reservoir. Thus, the apparatus may be compatible with at least three different flow control methods but not limited to these alternatives.

It should be noted that the number, arrangement, and interconnection of the constructive elements constituting the apparatus, which are shown in FIG. 1, are not intended to be any limitation of the present invention, but merely used to provide a general idea and some alternatives of how the constructive elements may be implemented within the apparatus. For example, the cell culture plate may be replaced with two or more cell culture plates, and the flow driving unit may be replaced with two or more flow driving units each configured to control a culture medium flow in one of the cell culture plates.

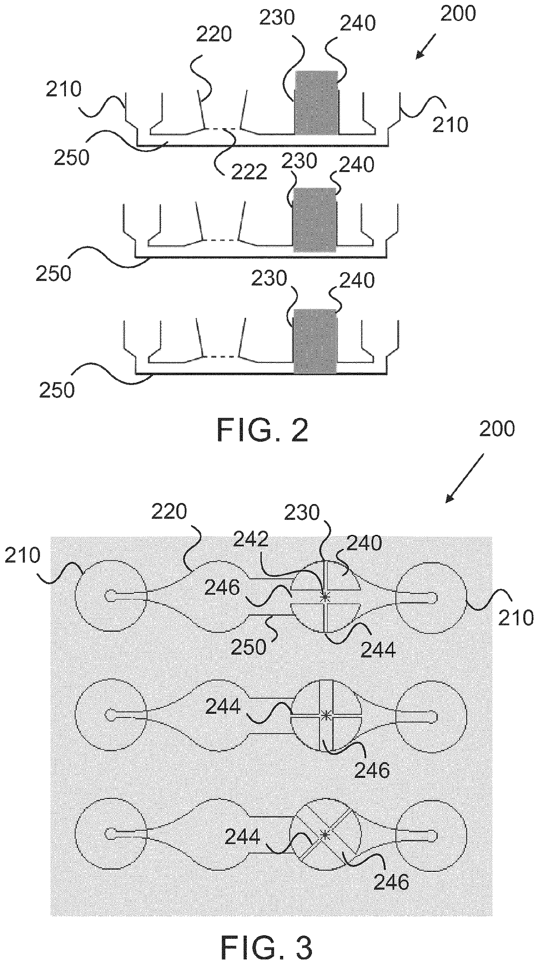

Figure 2 shows an example of a cell culture module 200 in a side view for various states. The apparatus may comprise multiple such modules, as indicated above. The module comprises two or more culture medium reservoirs 210 (also "the reservoirs") connected by a microfluidic channel 250 including a first culture medium reservoir and a second culture medium reservoir. The reservoirs can be configured for facilitating provision of cell culture medium to the microfluidic channel. Each or both of the first and the second reservoirs may be provided as a well, such as an open-top well or a closed-top well. The first and/or the second culture medium reservoir may serve as an end point for the microfluidic channel but it could also be possible to situate them along the microfluidic channel so that the corresponding reservoir has two outlets to the microfluidic channel.

The culture medium reservoir(s) may have a top part with an inlet for the culture medium and a bottom part with an outlet for the culture medium. The first and/or second culture medium reservoirs may be implemented as a hollow tube. They may be identical. The first and/or second culture medium reservoir may have a circular, polygonal or oval cross-section, for example. The bottom part of the first and/or second culture medium reservoir may have a tapered profile, narrowing towards the microfluidic channel.

The microfluidic channel of the cell culture module may have a variable or constant channel height. It may also have a variable or constant channel width. It may be shaped and sized for achieving required flow characteristics (e.g., flow rate).

The module 200 also comprises two or more open-top wells including a first open-top well 220 and a second open-top well 230. The microfluidic channel 250 extends through these open-top wells to form a fluid connection. The microfluidic channel can thus comprise multiple partial flow channels separated by the bottom regions of the open-top wells to form a continuous fluid connection from the first reservoir to the second reservoir. The first and/or the second reservoir may serve as an end point for the fluid connection but also as a point or points along the fluid connection, as indicated above. The fluid connection is such that it facilitates cell cultivation in one or more of the open-top wells, in particular in the first open-top well.

Any or all of the open-top wells, in particular the first open-top well, may be configured for cell cultivation. Any or all of the open-top wells, in particular those configured for cell cultivation, may comprise two chambers: an upper (apical) chamber and a lower (basal) chamber. The module may be configured for the fluid connection to go through the basal chamber arranged under the apical chamber. The apical and basal chambers may be separated by a separating surface 222 such as a membrane. The separating surface may have through-pores. It may be arranged between the apical and basal chamber so that the chambers are in flow communication with each other, for example via the through-pores. Flow of culture medium may be facilitated between the culture medium reservoirs in the cell culture module, for example by placing the apparatus on a rocking platform or connecting the apparatus to a pneumatic pump. This may be done, for example, with the flow driving unit. The apparatus thus configured may facilitate cultivation of cells of the same or different types on the basal surface of the separating surface in the cell culture module(s), i.e., on the surface on the side of the basal chamber. This may be done under uniform and controlled in vitro conditions. The deposition of the cells on the basal surface of the separating surface may be provided without having to invert the apparatus or use high concentrations of the cells in the culture medium. In one example, the basal chamber has a tapered profile narrowing towards the microfluidic channel. The apical chamber may, for example, have a straight profile so that its cross section remains constant across the longitudinal (height) dimension of the chamber and the open-top well.

Any or all of the open-top wells, in particular the first and the second open-top well, may be made small, for example less than 10 millimeters in the longitudinal (height) dimension. As such, the total height of the one or more cell culture modules may be 10 millimeters or less.

Any or all of the open-top wells, in particular the first and the second open-top well, may be of the same shape and/or size, allowing them to be used interchangeably. However, they may also have a different shape and/or size. For example, one or more of the open-top wells, such as the first open-top well may be a tapered well, for example narrowing towards the microfluidic channel. On the other hand, one or more of the open-top wells, such as the second open-top well may be a straight well, which may protrude from the microfluidic channel with a constant cross-section, e.g. in a direction perpendicular to the microfluidic channel.

The second open-top well is configured to receive a flow restrictor 240 to at least partially block the fluid connection through the second open-top well. For this purpose, the flow restrictor may physically block, partially or fully, the fluid connection through the second open-top well. The apparatus may be configured for the flow restrictor to be moved translationally and/or rotationally for blocking the fluid connection. The flow restrictor may thus be configured to act as a physical barrier in the microfluidic channel so that it may, by itself, physically intersect the fluid connection and so that the degree to which it intersects the fluid connection is controllable, in particular by translational and/or rotational movement of the flow restrictor. The flow restrictor may thus function as a valve, such as a translational and/or rotational valve. The flow restrictor may be integral to the cell culture module or it may be a separate piece, such as a separate plug. A simple flow restrictor may be provided as a cylindrical plug. Other shapes are also possible so that a simple flow restrictor may be shaped not only as a cylinder but also as a rectangular prism, for example. The apparatus may be configured so that the flow restrictor may be removed or otherwise adjusted to open an ambient connection from the microfluidic channel through the corresponding open-top well, such as the second open-top well. This ambient connection may be an air connection.

The apparatus may be configured for the flow restrictor to have multiple positions for blocking the fluid connection. Here, "position" may indicate translational and/or rotational placement of the flow restrictor. It may have a blocking position, in which it completely blocks the fluid connection through the corresponding open-top well, such as the second open-top well. It may have a connecting position, in which it facilitates the fluid connection through the corresponding open-top well, such as the second open-top well, by only partially blocking the fluid connection or by not blocking the fluid connection at all. Correspondingly, it may have one or more partially blocking positions in which it facilitates the fluid connection through the corresponding open-top well, such as the second open-top well, by only partially blocking the fluid connection. The partially blocking positions may differ so that they facilitate different flow rates for the fluid connection through the corresponding open-top well. Finally, the flow restrictor may have a non-blocking position in which it facilitates the fluid connection through the corresponding open-top well, such as the second open-top well, by not blocking the fluid connection at all. For example, the apparatus may be configured for the flow restrictor to have a blocking position and one or more connecting positions, which may include a partially blocking position and/or a non-blocking position.

The flow restrictor may be provided as a plunger. For this purpose, the apparatus may be configured for the flow restrictor to be plugged into one or more open-top wells for at least partially blocking the fluid connection as described above. The plunger may be provided as a simple plug or as a more complicated construction. Importantly, it allows at least partially blocking the fluid connection by translational movement of the plunger within the corresponding open-top well, such as the second open-top well. For this purpose, the plunger may be moved perpendicularly with respect to the fluid connection through the corresponding open-top well, e.g. perpendicularly with respect to the microfluidic channel at the location of the well.

The flow restrictor may be a thoroughly solid object, e.g. a simply connected object in a topological sense, for at least partially blocking the fluid connection or it may have one or more through holes for facilitating the fluid connection through the flow restrictor. Alternatively or additionally, it may be configured for abutting against a wall of the microfluidic channel to form one or more channels for the fluid connection between the flow restrictor and the microfluidic channel. In other words, the apparatus is configured for the flow restrictor to provide one or more channels for the fluid connection, where the channels may be formed by one or more holes through the flow restrictor and/or between the flow restrictor and the microfluidic channel, e.g. one or more walls of the microfluidic channel. Each such channel may correspond to a connecting position of the one or more connecting positions, either a partially blocking position or a non-blocking position. For a partially blocking position, the channel may be narrower than the microfluidic channel at the location of the corresponding open-top well, such as the second open-top well. For a non-blocking position, the channel may be of equal cross-sectional size as the microfluidic channel at the location of the corresponding open-top well, such as the second open-top well. Any or all of the one or more channels may be straight channels, facilitating a straight fluid connection in the connecting position. The apparatus may be configured for any or all of the one or more channels to be rotatable parallel with respect to the orientation of the microfluid channel at the location of the corresponding open-top well, such as the second open-top well.

Figure 2 illustrates a situation, where the apparatus is configured for the flow restrictor 240, such as a plunger, to at least partially block the fluid connection by translational movement. In the uppermost example, the flow restrictor is in a non-blocking position, allowing the fluid connection to be formed through the second open-top well 230. In the middle example, the flow restrictor is repositioned translationally towards the bottom of the microfluidic channel 250 so that it only partially blocks the fluid connection, i.e. into a partially blocking position. In the lowermost example, the flow restrictor is repositioned translationally against the bottom of the microfluidic channel so that it completely blocks the fluid connection, i.e. into a blocking position.

The bottom of the microfluidic channel may be shaped to match the bottom of the flow restrictor at the location of the corresponding open-top well, such as the second open-top well. This allows preventing fluid connection between the bottom of the flow restrictor and the bottom of the microfluidic channel when the flow restrictor is positioned to at least partially block the fluid connection. When there are no through-holes or channels for the fluid connection, this allows also completely blocking the fluid connection. The bottom of the microfluidic channel may be flat, either across the whole length of the microfluidic channel from the first culture medium reservoir to the second culture medium reservoir or along one or more sections therebetween. The bottom may be flat for at least at the location of one or more open-top wells, such as the second open-top well, configured for receiving one or more flow restrictors. While the bottom may have differences in elevation along the microfluidic channel, it is noted that the apparatus may be configured for facilitating the flow even without such differences.

Any open-top well(s), such as the second open-top well, configured for receiving a flow restrictor may comprises a wall extending upward from the microfluidic channel, for example perpendicularly with respect to the microfluidic channel, or the fluid connection. The wall may have any cross-section, for example a rectangular cross section, such as a square cross section, or a circular cross section. The apparatus may be configured for the flow restrictor to be supported against the wall.

Instead or in addition to translational operation, the apparatus may also be configured for the flow restrictor to be actuated with rotational movement for at least partially blocking the fluid connection. The flow restrictor may have a rotation axis 242, for example in the longitudinal dimension of the flow restrictor. With the rotation axis perpendicular to the fluid connection through the corresponding open-top well, such as the second open-top well, the flow restrictor may be configured to rotate about the rotation axis between one or more of the positions indicated above. These may include any combination of one or more connecting positions and/or one or more blocking positions. The one or more connecting positions may include any combination of one or more partially blocking positions and/or one or more non-blocking positions.

Figure 3 shows an example of a cell culture module 200 in a top-down view for various states. It illustrates a situation, where the apparatus is configured for the flow restrictor 240 to block, partially or fully, the fluid connection, at least, by rotational movement. In the uppermost example, the flow restrictor is in a first partially blocking position, allowing the fluid connection to be formed through the second open-top well 230. The fluid connection is facilitated by a first channel 246 through the flow restrictor, which is thus in the first connecting position. As an alternative, the first channel could also be wider to have the flow restrictor in a non-blocking position. In the middle example, the flow restrictor is repositioned rotationally into the second connecting position so that the fluid connection is formed by a second channel 244 through the flow restrictor. In the illustrated example, the fluid connection is only formed by one channel at a time. In general, the apparatus may be configured so that the fluid connection is formed only by one channel at a time, by one or more channels at a time or only by two or more channels at a time. The second channel is narrower than the first channer, facilitating a flow rate for the fluid connection in the second connecting position that is smaller than the flow rate in the first connecting position. In the lowermost example, the flow restrictor is repositioned rotationally so that it completely blocks the fluid connection, i.e. into a blocking position. This may be achieved when a wall of the flow restrictor completely obstructs the microfluidic channel.

As illustrated, in a specific example the flow restrictor may be shaped to form two or more channels for facilitating the fluid connection, which channels may be straight channels. In particular, two channels may be perpendicular with respect to each other. The two or more channels may be rotatable parallel with respect to the orientation of the microfluid channel at the location of the corresponding open-top well, such as the second open-top well.

The number of open-top wells of the cell culture module may be larger than two, for example three, four or more. However, the number may easily be also much larger, for example 10 or more, or 48 or more. In particular, this means that the number of open-top wells configured for cell cultivation may be two or more. Alternatively or additionally, the number of open-top wells for a flow restrictor may be two or more.

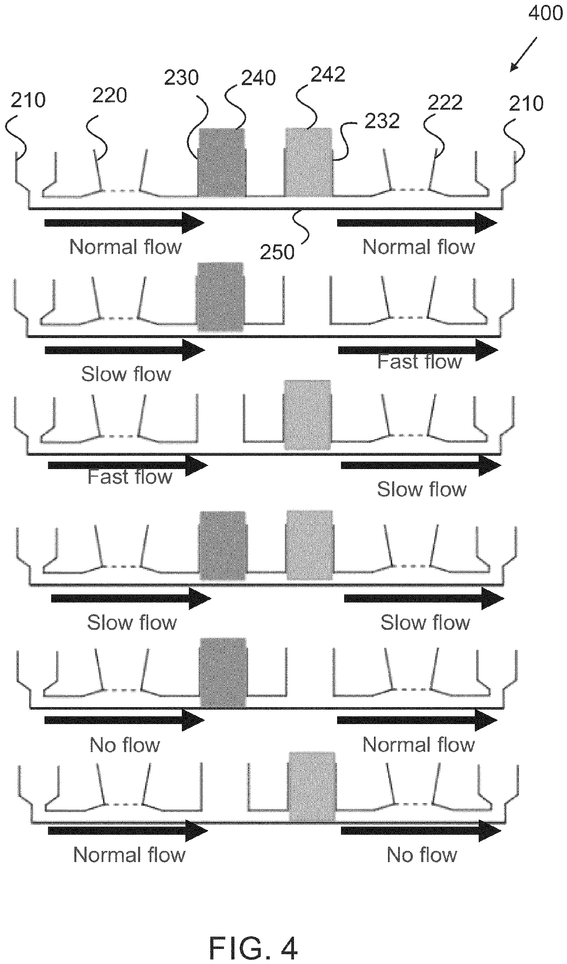

Figure 4 shows an example of a cell culture module in a side view for various states. The cell culture module can be in accordance of any of the examples above. However, in addition to the first open-top well 220 and the second-open top well 220, it comprises two or more additional open-top wells between the first and the second culture medium reservoirs 210 through which wells the fluid connection may be formed. In particular, it may comprise a third open-top well 222 configured for cell cultivation, which may be of the same size and/or shape as the first open-top well 220, but also different. It may also comprise a fourth open-top well 232, which may be of the same size and/or shape as the third open-top well 230, but also different. The first and the third open-top wells can be positioned between the first culture medium reservoir and the second culture medium reservoir for the fluid connection, whereas the second open-top well and the fourth open-top well may then be positioned between the first open-top well and the third open-top well for the fluid connection. The order of the wells and reservoirs along the microfluidic channel may thus be as follows: the first culture medium reservoir, the first open-top well, the second open-top well, the third open-top well, the fourth open-top well, the second culture medium reservoir. In absence of flow restrictors in any of the wells, there may thus be a fluid connection from the first culture medium reservoir to the second culture medium reservoir via the microfluidic channel through the four open-top wells.

Importantly, the fourth open-top well may also be configured to receive a flow restrictor to at least partially block the fluid connection through the fourth open-top well. The flow restrictor may be in accordance with any of the examples as described above. It may be identical to the flow restrictor for the second open-top well or different. In particular, it may have the same shape and/or size as a flow restrictor for the second open-top well. A single flow restrictor may be used for both the second and the third open-top well or two separate flow restrictors may be used, in which case they may be interchangeable or non-interchangeable.

With two-open top wells for the flow restrictor(s), fluid flow through the first open-top well and the third open-top well can be flexibly controlled. In particular, different flow rates may be provided for the two wells for cell cultivation in the same cell culture module. Figure 4 illustrates various states for the flow as is detailed below starting from the uppermost example and proceeding downwards until the lowermost example. In the first example, both the second and the fourth open-top wells 230, 240 have a flow restrictor therein, which may be referred as the first flow restrictor 232 and the second flow restrictor 242, respectively. They may both be in a non-blocking state so that the flow through both the second and the fourth open-top wells is at a first rate, which may be the same or different through the second open-top well and the fourth open-top well. This may be termed "normal flow". In the second example, the first flow restrictor is positioned in the second open-top well into a partially blocking position, whereas the fourth open-top well may be opened with an ambient connection. The second flow restrictor may be removed from the fourth open-top well or adjusted accordingly. As a result, flow through the first open-top well is reduced (to a "slow flow") to whereas flow through the third open-top well may be increased (to a "fast flow"). In converse, and in accordance with the third example, the first or the second flow restrictor may be positioned in the fourth open-top well into a partially blocking position, whereas the second open-top well may be opened with an ambient connection. The first flow restrictor may thus be removed from the second open-top well or adjusted accordingly. As a result, flow through the third open-top well is reduced (to a "slow flow") to whereas flow through the first open-top well may be increased (to a "fast flow"). In both cases, the slow flow may be slower than the fast flow but also that of the normal flow. In converse the fast flow may be faster than the normal flow.

In a fourth example, both the first flow restrictor and the second flow restrictor are positioned into a partially blocking position, in the second open-top well and in the fourth open-top well, respectively. As a result, flow through both the first and the third open-top well is reduced (to a "slow flow"). In the fifth example, the first flow restrictor is positioned into a blocking position in the second open-top well. As a result, there is no flow through the first open-top well. However, the flow through the third open-top well may still proceed (with "normal flow"). For this, the fourth open-top well may be utilized as a culture medium reservoir. The same holds in converse for the second open-top well, as illustrated in the sixth and final example. There, the first or second flow restrictor is positioned into a blocking position in the fourth open-top well. As a result, there is no flow through the third open-top well. However, the flow through the first open-top well may now proceed (with "normal flow"). For this, the second open-top well may be utilized as a culture medium reservoir.

The apparatus may be configured for the first and/or the second flow restrictor to be moved translationally and/or rotationally for blocking, partially or fully, the fluid connection. In particular, the first flow restrictor may be of the same or different type in comparison to the second flow restrictor with respect to its operation: translational and/or rotational. For example, one of the flow restrictors may be operated by translation and the other by rotation for blocking the fluid connection.

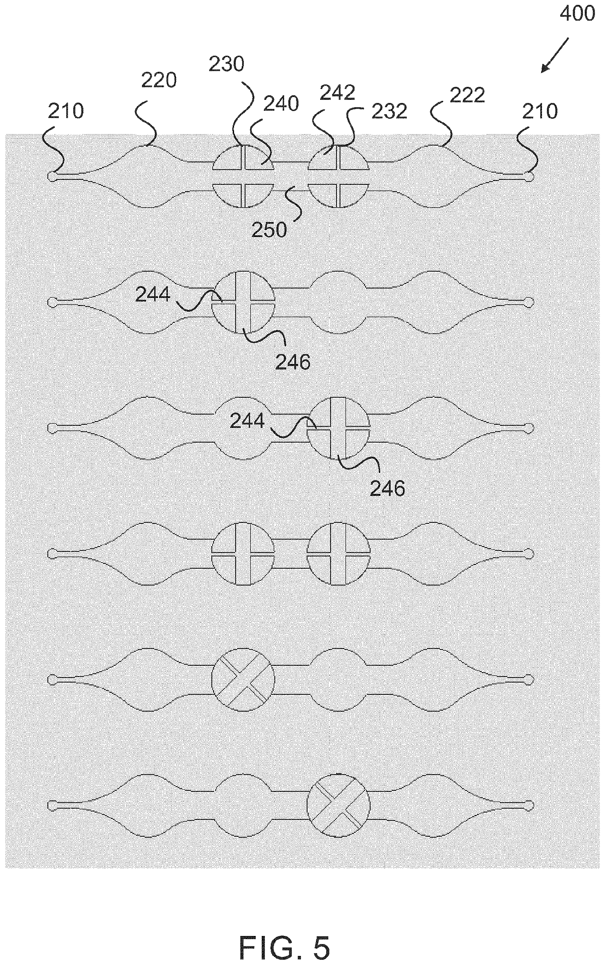

Figure 5 shows an example of a cell culture module in a top-down view for various states. In this case, the apparatus is configured for the first and the second flow restrictor to be moved, at least, rotationally for blocking, partially or fully, the fluid connection. The examples are analogous to those presented with reference to figure 4 and in the same order. As a slight difference, the connecting positions in the first (topmost) example are partially blocking positions but the apparatus may well be configured for these to be non-blocking positions or a combination of a partially blocking position and a non-blocking position as well. The first channel 246 and the second channel 244 can be as presented with reference to Figure 3. In particular, both the first and the second flow restrictor may comprise such channels. They may be of the same or different size for the two flow restrictors.

In any of the embodiments implied above, one or more of the open-top wells, in particular the second open-top well and/or the fourth open-top well, may thus be configured as valve seats for the flow restrictor. The flow restrictor may then serve as a valve stem compatible with one or more open-top wells, in particular with the second open-top well and/or the fourth open-top well, for at least partially blocking the fluid connection through the corresponding open-top well. The flow restrictor(s) within the open-top well(s) may thus act as passive valves in the sense that no external energy is required to maintain their state. External energy may still be used for changing the state of such valve by adjusting the position of the flow restrictor(s). Whatever is stated with reference to any open-top well for a flow restrictor, such as the second open-top well, may be applied also to the fourth open-top well. The first and/or second flow restrictor may be part of the apparatus or provided separately. In general, the second and/or fourth open-top well may be configured for utilization as a culture medium reservoir.

Any or all of the culture medium reservoirs and the open-top wells may be aligned with parallel longitudinal axes. The longitudinal axis of any or all of these may be perpendicular with respect to the microfluidic channel.

The fluid connection between the first and the second culture medium reservoir may be linear, as illustrated in the two leftmost columns of cell culture modules 200, 400 in Figure 1. Alternatively, it may form a meandering pattern, as illustrated in the rightmost column of cell culture modules 106 in Figure 1. In particular, the pattern may be sawtooth pattern with sharp and/or rounded corners. The open-top wells, such as the first, second, third and fourth open-top well, may be located at the corners of the sawtooth pattern. The apparatus may comprise cell culture modules only in linear pattern, only in meandering pattern or both in linear and meandering pattern. In a single cell culture module, the distance between any or all adjacent culture medium reservoirs and/or open-top wells may be equal.

The apparatus 100 may also comprise a pipetting station, in which case the apparatus as a whole may be termed a cell culture incubator. The pipetting station can be configured to feed the culture medium to either or both of the culture medium reservoirs of any or all of the cell culture modules. The apparatus may comprise one or more sensors for measurements, for example at any or all of the open-top wells and/or culture medium reservoirs.

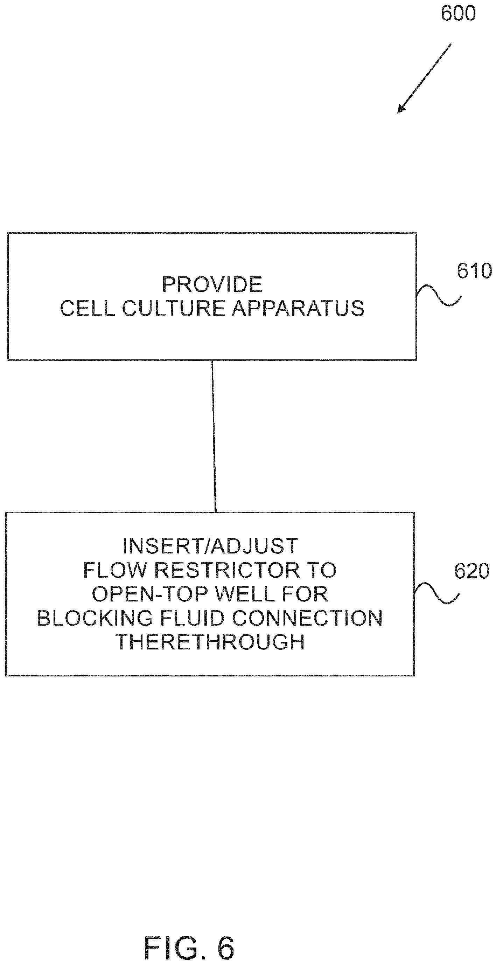

Figure 6 shows an example of a method 600, which may be used for facilitating cell cultivation. The method comprises providing 610 a cell culture apparatus comprising one or more cell culture modules. The one or more cell culture modules comprise a first culture medium reservoir, a second culture medium reservoir, a first open-top well configured for cell cultivation, a second open-top well anda microfluidic channel configured to provide a fluid connection from the first culture medium reservoir to the second culture medium reservoir through the first open-top well and the second open-top well. The apparatus may be in accordance with any of the examples presented in this disclosure. The method also comprises inserting and/or adjusting a flow restrictor, at least, to the second open-top well for at least partially blocking the fluid connection through the second open-top well. The flow restrictor may be in accordance with any of the examples presented in this disclosure. The flow restrictor may be inserted by plugging it into the open-top well. It may be adjusted by translational and/or rotational movement.

The different functions discussed herein may be performed in a different order and/or concurrently with each other.

Any range or device value given herein may be extended or altered without losing the effect sought, unless indicated otherwise. Also any example may be combined with another example unless explicitly disallowed.

Although the subject matter has been described in language specific to structural features and/or acts, it is to be understood that the subject matter defined in the appended claims is not necessarily limited to the specific features or acts described above. Rather, the specific features and acts described above are disclosed as examples of implementing the claims and other equivalent features and acts are intended to be within the scope of the claims.

It will be understood that the benefits and advantages described above may relate to one embodiment or may relate to several embodiments. The embodiments are not limited to those that solve any or all of the stated problems or those that have any or all of the stated benefits and advantages. It will further be understood that reference to 'an' item may refer to one or more of those items.

The term 'comprising' is used herein to mean including the method, blocks or elements identified, but that such blocks or elements do not comprise an exclusive list and a method or apparatus may contain additional blocks or elements.

Numerical descriptors such as 'first', 'second', and the like are used in this text simply as a way of differentiating between parts that otherwise have similar names. The numerical descriptors are not to be construed as indicating any particular order, such as an order of preference, manufacture, or occurrence in any particular structure.

Expressions such as 'plurality' are in this text to indicate that the entities referred thereby are in plural, i.e. the number of the entities is two or more.

Although the invention has been described in conjunction with a certain type of apparatus and/or method, it should be understood that the invention is not limited to any certain type of apparatus and/or method. While the present inventions have been described in connection with a number of examples, embodiments and implementations, the present inventions are not so limited, but rather cover various modifications, and equivalent arrangements, which fall within the purview of the claims. Although various examples have been described above with a certain degree of particularity, or with reference to one or more individual embodiments, those skilled in the art could make numerous alterations to the disclosed examples without departing from the scope of this specification.

## Claims

1. A cell culture apparatus (100) comprising one or more cell culture modules, the one or more cell culture modules comprising:
- a first culture medium reservoir;
- a second culture medium reservoir;
- a first open-top well configured for cell cultivation;
- a second open-top well; and a
- a microfluidic channel configured to provide a fluid connection from the first culture medium reservoir to the second culture medium reservoir through the first open-top well and the second open-top well;
wherein the second open-top well is configured to receive a flow restrictor to at least partially block the fluid connection through the second open-top well.

2. The apparatus according to claim 1, wherein the apparatus is configured for the flow restrictor, in a blocking position, to completely block the fluid connection through the second open-top well and, in a connecting position, facilitate the fluid connection through the second open-top well.

3. The apparatus according to claim 1 or 2, wherein the apparatus is configured for the flow restrictor, in a first connecting position, to facilitate the fluid connection through the second open-top well with a first flow rate and, in a second connecting position, facilitate the fluid connection through the second open-top well with a second flow rate, the second flow rate being smaller than the first.

4. The apparatus according to any preceding claim, wherein the flow restrictor is a plunger.

5. The apparatus according to claim 4, configured for the plunger to be moved perpendicularly with respect to the fluid connection through the second open-top well for at least partially blocking the fluid connection.

6. The apparatus according to any preceding claim, wherein the flow restrictor has a rotation axis and, with the rotation axis perpendicular to the fluid connection through the second open-top well, is configured to rotate about the rotation axis between a first connecting position and a second connecting position and/or a blocking position for the fluid connection.

7. The apparatus according to any preceding claim, comprising a third open-top well configured for cell cultivation and a fourth open-top well, the third open-top well being positioned between the first culture medium reservoir and the second culture medium reservoir for the fluid connection and the second open-top well and the fourth open-top well being positioned between the first open-top well and the third open-top well for the fluid connection, wherein the fourth open-top well is configured to receive the flow restrictor to at least partially block the fluid connection through the fourth open-top well.

8. The apparatus according to claim 7, comprising a first and a second flow restrictor, the apparatus being configured for the second flow restrictor to at least partially block the fluid connection through the fourth open-top well while the first flow restrictor at least partially blocks the fluid connection through the second open-top well.

9. The apparatus according to any preceding claim, comprising a third open-top well configured for cell cultivation and a fourth open-top well, the third open-top well being positioned between the first culture medium reservoir and the second culture medium reservoir for the fluid connection and the second open-top well and the fourth open-top well being positioned between the first open-top well and the third open-top well for the fluid connection, wherein the fourth open-top well is configured to receive a second flow restrictor to at least partially block the fluid connection through the fourth open-top well, the flow restrictor and the second flow restrictor being non-interchangeable.

10. The apparatus according to any preceding claim, wherein the bottom of the microfluidic channel is shaped to match the bottom of the flow restrictor at the location of the second open-top well for preventing fluid connection below the bottom of the flow restrictor when the flow restrictor is positioned to at least partially block the fluid connection.

11. The apparatus according to claim 10, wherein the bottom of the microfluidic channel, at least at the location of the second open-top well, is flat.

12. The apparatus according to any preceding claim, wherein the second open-top well comprises a wall extending upward from the microfluidic channel and the apparatus is configured for the flow restrictor to be supported against the wall.

13. The apparatus according to any preceding claim, wherein the fluid connection between the first and the second culture medium reservoir is linear.

14. The apparatus according to any of claims 1-12, wherein the fluid connection between the first and the second culture medium reservoir forms a meandering pattern.

15. The apparatus according to any preceding claim, wherein the distance between any or all adjacent culture medium reservoirs and/or open-top wells is equal.

16. A method comprising providing a cell culture apparatus comprising one or more cell culture modules, the one or more cell culture modules comprising:
- a first culture medium reservoir;
- a second culture medium reservoir;
- a first open-top well configured for cell cultivation;
- a second open-top well; and
- a microfluidic channel configured to provide a fluid connection from the first culture medium reservoir to the second culture medium reservoir through the first open-top well and the second open-top well;
and inserting and/or adjusting a flow restrictor, at least, to the second open-top well for at least partially blocking the fluid connection through the second open-top well.
